# EUROPÄISCHE PATENTSCHRIFT

(11) **EP 1 385 519 B1**
(45) Veröffentlichungstag und Bekanntmachung des Hinweises auf die Patenterteilung: **30.08.2006**
(21) Anmeldenummer: 02719914.0
(22) Anmeldetag: 26.02.2002
(51) Int. Cl.: A61K 31/46, A61P 29/00

(54) **TIOTROPIUMSALZE ZUR BEHANDLUNG VON ENTZÜNDLICHEN ERKRANKUNGEN**
COMPOUNDS FOR TREATING INFLAMMATORY DISEASES
COMPOSES DESTINES AU TRAITEMENT DE MALADIES INFLAMMATOIRES

(30) Priorität: 13.03.2001 DE 10111843
(43) Veröffentlichungstag der Anmeldung: 04.02.2004
(60) Teilanmeldung: 06114951.4
(73) Patentinhaber: Boehringer Ingelheim Pharma GmbH & Co.KG, 55216 Ingelheim am Rhein (DE)
(72) Erfinder: DISSE, Bernd, 55127 Mainz am Rhein (DE)
(86) Internationale Anmeldenummer: PCT/EP2002/001986
(87) Internationale Veröffentlichungsnummer: WO 2002/072095

(56) Entgegenhaltungen:
- WO-A-00/47200
- WO-A-01/10427
- WO-A-01/12167
- WO-A-01/78739
- WO-A-99/66944
- US-A- 6 150 418
- WITEK T J JR: "Anticholinergic bronchodilators." RESPIRATORY CARE CLINICS OF NORTH AMERICA. UNITED STATES DEC 1999, Bd. 5, Nr. 4, Dezember 1999 (1999-12), Seiten 521-536, XP008014468 ISSN: 1078-5337
- LITTNER M R ET AL: "LONG-ACTING BRONCHODILATION WITH ONCE-DAILY DOSING OF TIOTROPIUM (SPIRIVA) IN STABLE CHRONIC OBSTRUCTIVE PULMONARY DISEASE" AMERICAN JOURNAL OF RESPIRATORY AND CRITICAL CARE MEDICINE, AMERICAN LUNG ASSOCIATION, NEW YORK, NY, US, Bd. 161, Nr. 4I, 2000, Seiten 1136-1142, XP001148373 ISSN: 1073-449X
- LASSERSON T ET AL: "Anticholinergic therapy for bronchiectasis." COCHRANE DATABASE OF SYSTEMATIC REVIEWS (ONLINE: UPDATE SOFTWARE) ENGLAND 2001, Nr. 4, 2001, Seite CD002163 XP008019810 ISSN: 1469-493X
- ROGERS D F: "Mucus pathophysiology in COPD: Differences to asthma, and pharmacotherapy" MONALDI ARCHIVES FOR CHEST DISEASE 2000 ITALY, Bd. 55, Nr. 4, 2000, Seiten 324-332, XP008019816 ISSN: 1122-0643

## Beschreibung

Die Erfindung betrifft die Verwendung von (1α,2β,4β,5α,7β)-7-[(Hydroxydi-2-thienylacetyl)oxy]-9,9-dimethyl-3-oxa-9-azoniatricyclo[3.3.1.0^{2,4}] nonane-salzen zur Herstellung eines Arzneimittels zur Vorbeugung und Behandlung von zystischer Fibrose, idiopatischer Lungenfibrose oder fibrosierender Alveolitis. Antimuscarinika, die in klinischer Praxis auch häufig als Anticholinergika bezeichnet werden, sind beispislweise bei der Therapie von Erkrankungen der Atemwege fest etabliert. Beispielsweise gehört die inhalative Applikation von Ipratropiumbromid (Atrovent®) als Bronchodilator häufig als fester Therapiebestandteil zur Behandlung von COPD, ein Begriff, der im folgenden für die verwandten Syndrome chronische Bronchitis, chronisch obstruktive Bronchitis und Lungenemphysem verwendet wird. Auch bei der Therapie von Asthma, gelangen Anticholinergika aufgrund ihrer bronchodilatorischen Wirkung zur Anwendung.

Die Verbindung (1α,2β,4β,5α,7β)-7-[(Hydroxydi-2-thienylacetyt)oxy]-9,9-dimethyl-3-oxa-g-azoniatricyclo[3.3.1.0^{2,4}]inonane-bromid, ist aus der Europäischen. Patentanmeldung EP 418 716 A1 bekannt und weist die folgende chemische Struktur auf: 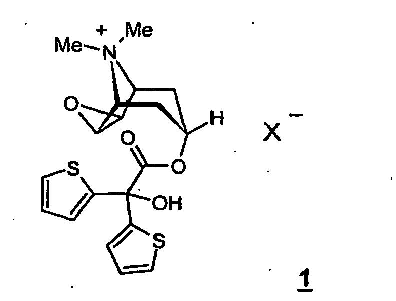 wobei X Bromid bedeutet. Die Bezeichnung Tiotropium ist im Rahmen der vorliegenden Erfindung als Bezugnahme auf das freie Kation (1') zu verstehen.

Tiotropiumbromid, wie auch andere Salze des Tiotropiums, sind als hochwirksame anticholinerge Bronchodilatoren bekannt und können deshalb bei der Therapie von Asthma oder COPD einen therapeutischen Nutzen entfalten. Siehe Am. J. Respir. Crit. Care Med. Vol. 161, pp 1136-1142, 2000 (D1)

Die Applikation von Tiotropiumsalzen 1 erfolgt vorzugsweise auf inhalativern Wege. Hierbei können geeignete Inhaltionspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten. Die inhalative Applikation kann ferner mittels geeigneter Lösungen des Tiotropiumsalzes 1 erfolgen.

Überraschenderweise wurde nun gefunden, daß die antimuskarinisch wirksamen Tiotropiumsalze 1 nicht nur als Bronchodilatoren bei Erkrankungen wie beispielsweise COPD wirkungsvoll Verwendung finden können, sondern ferner durch eine anti-inflammatorische Wirksamkeit gekennzeichnt sind. Diese beruht darauf, daß die Freisetzung von entzündlichen Mediatoren wie 15-HETE aus Epithelzellen, wie Histamin, Leukotriene und Tryptase aus Mastzellen, chemotaktische Aktivität (für neutrophile Granulozyten, eosinophile Granulozyten und Makrophagen) wie LTB4 und Interleukin 8 aus Alveolarmakrophagen durch Acetylcholin bewirkt oder gefördet und durch Tiotropium gehemmt wird. Überraschenderweise wurde gefunden, daß dieses theoretisch allen antimuskarinischen Wirkstoffen zuzurechnende Potential erst in Verbindung mit einem sehr langsam vom Rezeptor abdissoziierenden Wirkstoff wie Tiotropium zur Geltung kommt, da chemotaktische und entzündliche Aktivität dauerhaft und nicht nur intermittierend abgeschaltet sein muß, um zu biologischer und klinischer Wirksamkeit zu gelangen.

Die Erfindung betrifft daher die Verwendung von Tiotropiumsalzen 1 zur Herstellung eines Arzneimittels zur Vorbeugung oder Behandlung von zystischer Fibrose, idiopatischer Lungenfibrose oder fibrosierender Alveolitis.

Bevorzugt betrifft die vorliegende Erfindung die Verwendung von Tiotropiumsalzen 1 zur Herstellung eines Arzneimittels zur Behandlung der entzündlichen Komponente bei Erkrankungen der oberen und unteren Atmungsorgane einschließlich der Lunge zystische Fibrose, idiopathische Lungenfibrose, fibrosierende Alveolitis.

Bevorzugt ist die Verwendung von Tiotropiumsalzen 1 zur Behandlung von Entzündungen in Verbindung mit dem anderen pulmonale Erkrankungen zystische Fibrose und fibrosierende Alveolitis.

Unter den im Rahmen der vorliegenden Erfindung einsetzbaren Tiotropiumsalzen 1 sind die Verbindungen zu verstehen, die neben Tiotropium als Gegenion (Anion) Chlorid, Bromid, lodid, Methansulfonat, para-Toluolsulfonat oder Methylsulfat enthalten. Im Rahmen der vorliegenden Erfindung sind von allen Tiotropiumsalzen das Methansulfonat, Chlorid, Bromid oder lodid bevorzugt, wobei dem Methansulfonat oder dem Bromid besondere Bedeutung zukommt. Von erfindungsgemäß herausragender Bedeutung ist das Tiotropiumbromid.

Ein weiterer Aspekt der vorliegenden Erfindung betrifft pharmazeutische Zubereitungen zur Behandlung der vorstehend genannten Erkrankungen. Diese können, ohne den Umfang der Erfindung darauf zu beschränken, Tiotropium 1' in solchen Mengen enthalten, daß pro Einmalgabe 0,1 - 80µg, bevorzugt 0,5 - 60 µg, besonders bevorzugt etwa 1 - 50µg enthalten sind. Beispielsweise und ohne den Gegenstand der vorliegenden Erfindung darauf zu beschränken, können pro Einmalgabe 2,5µg, 5µg, 10µg, 18µg, 20µg, 36µg oder 40µg **1'** appliziert werden.

Wird als erfindungsgemäß bevorzugtes Tiotropiumsalz 1 Tiotropiumbromid verwendet, entsprechen die vorstehend beispielhaft genannten pro Einmalgabe applizierten Wirkstoffmengen von 1' den nachfolgenden pro Einmalgabe applizierten Mengen an 1: 3µg, 6µg, 12µg, 21,7µg, 24,1 µg, 43,3µg und 48,1 µg 1.

Die erfindungsgemäße Verwendung von Tiotropiumsalzen 1 schließt die Verwendung der dadurch gebildeten Solvate und Hydrate, bevorzugt der Hydrate, besonders bevorzugt der Monohydrate, mit ein.

Wird als erfindungsgemäß bevorzugtes Tiotropiumsalz 1 beispielsweise das Tiotropiumbromidmonohydrat eingesetzt, entsprechen die vorstehend beispielhaft genannten pro Einmalgabe applizierten Wirkstoffmengen von 1' den nachfolgenden pro Einmalgabe applizierten Mengen an 1 (Monohydrat): 3,1 µg, 6,2µg, 12,5µg, 22,5µg, 25µg, 45µg und 50µg.

Die Applikation der Tiotropiumsalze 1 im Rahmen der erfindungsgemäßen Verwendung erfolgt bevorzugt auf inhalativem Wege. Hierzu müssen die Tiotropiumsalze 1 in inhalierbaren Darreichungsformen bereitgestellt werden. Als inlialierbare Darreichungsformen kommen Inhalationspulver, treibgashaltige Dosieraerosole oder treibgasfreie Inhalationslösungen in Betracht. Erfindungsgemäße Inhalationspulver enthaltend die Tiotropiumsalze 1 gegebenenfalls im Gemisch mit physiologisch verträglichen Hilfsstoffen. Im Rahmen der vorliegenden Erfindung sind von dem Begriff treibgasfreie Inhaltionslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

### A) Inhalationspulver:

Die im Rahmen der erfindungsgemäßen Verwendung einsetzbaren Inhaltionspulver können 1 entweder allein oder im Gemisch mit geeigneten physiologisch unbedenkliche-N Hilfsstoffen enthalten.
Sind die Tiotropiumsalze 1 im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sirine der Erfindung gelangt Lactose, höchst bevorzugt Lactosemonohydrat als Hilfsstoff zur Anwendung.

Die Hilfsstoffe weisen im Rahmen der erfindungsgemäßen Inhaltionspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfihdungsgemäßen Inhaltionspulver mikronisierter Wirkstoff 1 , vorzugsweise mit einer mittleren Teilchengröße von 0,5 bis 10µm, besonders bevorzugt von 1 bis 6µm, der Hilfsstoffmischung beigemischt. Verfahren zur Herstellung der erfindungsgemäßen Inhaltionspulver durch Mahlen und Mikronisieren sowie durch abschließendes Mischen der Bestandteile sind aus dem Stand der Technik bekannt.

Erfindungsgemäße Inhalationspulver, die neben 1 ferner einen physiologisch unbedenklichen Hilfsstoff enthalten, können beispielsweise mittels Inhalatoren appliziert werden, die eine einzelne Dosis aus einem Vorrat mittels einer Meßkammer, wie er in der US 4570630A beschrieben wird, oder über andere apparative Vorrichtungen, wie sie in der DE 36 25 685 A beschrieben werden, dosieren. Die erfindungsgemäßen Inhalationspulver, die 1 gegebenenfalls in Verbindung mit einem physiologisch verträglichen Hilfsstoff enthalten, können beispielsweise mittels des unter dem Namen Turbuhaler® bekannten Inhalators beziehungsweise mit Inhalatoren wie sie beispielsweise in der EP 237507 A offenbart werden, appliziert werden. Vorzugsweise werden die erfindungsgemäßen Inhalationspulver, die neben 1 physiologisch unbedenklichen Hilfsstoff enthalten, in Kapseln abgefüllt (zu sogenannten Inhaletten), die in Inhalatoren wie beispielsweise in der WO 94/28958 beschrieben, zur Anwendung gelangen.

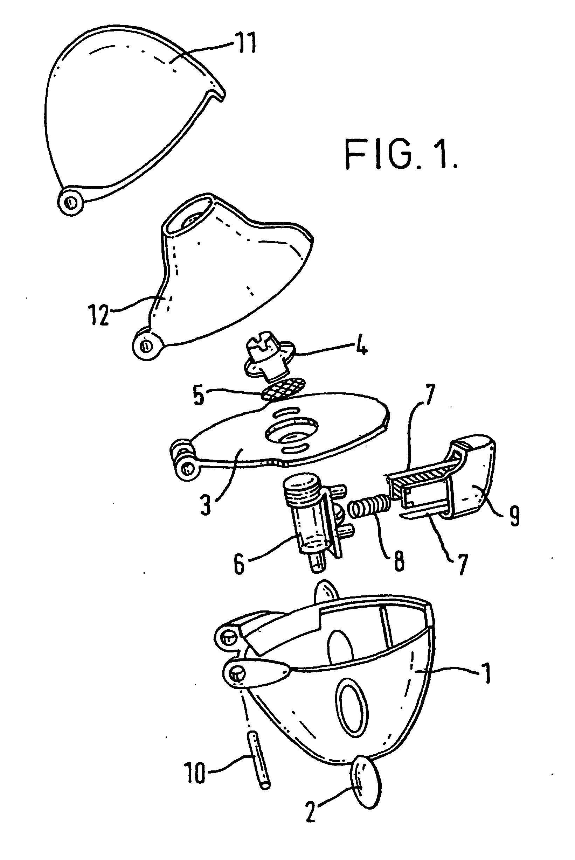

Ein zur Anwendung der erfindungsgemäßen Arzneimittelkombination in Inhaletten besonders bevorzugter Inhalator ist Figur 1 zu entnehmen.
Dieser Inhalator (Handyhaler) für die Inhalation pulverförmiger Arzneimittel aus Kapseln ist gekennzeichnet durch ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, sowie ein über eine Achse 10 klappbar mit dem Gehäuse 1, dem Deck 3 und einer Kappe 11 verbundenes Mundstück 12.

Sollen die erfindungsgemäßen Inhalationspulver im Sinne der vorstehend gennannten bevorzugten Anwendung in Kapseln (Inhaletten) abgefüllt werden, bieten sich Füllmengen von 1 bis 30mg, bevorzugt von 3 bis 20mg, bevorzugt 5 bis 10 mg Inhalationspulver pro Kapsel an. Diese enthalten efindungsgemäß die bereits vorstehend für 1' genannten Dosierungen pro Einmalgabe.

### B) Treibgashaltige Inhalationsaerosole:

Die im Rahmen der erfindungsgemäßen Verwendung einsetzbaren treibgashaltigen Inhaltionsaerosole können 1 im Treibgas gelöst oder in dispergierter Form enthalten. Die zur Herstellung der Inhaltionsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind fluorierte Alkanderivate ausgewählt aus TG134a (1,1,1,2-Tetrafluorethan), TG227 (1,1,1,2,3,3,3-Heptafluorpropan) und Mischungen derselben.

Die im Rahmen der erfindungsgemäßen Verwendung einsetzbaren treibgasnaltigen Inhaltionsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die im Rahmen der erfindungsgemäßen Verwendung einsetzbaren treibgashaltigen Inhaltionsaerosole können bis zu 5 Gew-% an Wirkstoff 1 enthalten. Die im Rahmen der erfindungsgemäßen Verwendung einsetzbaren treibgashaltigen enthalten beispielsweise 0,002 bis 5 Gew-%, 0,01 bis 3 Gew-%, 0,015 bis 2 Gew-% an Wirkstoff 1.

Liegen die Wirkstoffe 1 in dispergierter Form vor, weisen die Wirkstoffteilchen bevorzugt eine mittlere Teilchengröße von bis zu 10 µm, bevorzugt von 0,1 bis 5 µm, besonders bevorzugt von 1 bis 5 µm auf.

Die im Rahmen der erfindungsgemäßen Verwendung einsetzbaren treibgashaltigen Inhaltionsaerosole können mittels im Stand der Technik bekannten Inhalatoren (MDIs = metered dose inhalers) appliziert werden. Dementsprechend betrifft ein weiterer Aspekt der vorliegenden Erfindung die erfindungsgemäße Verwendung von 1 zur Herstellung von Arzneimitteln in Form von wie vorstehend beschriebenen treibgashaltigen Aerosolen in Verbindung mit einem oder mehreren zur Verabreichung dieser Aerosole geeigneten Inhalatoren.

Die vorliegende Erfindung betrifft ferner die erfindungsgemäße Verwendung von 1 zur Herstellung von Kartuschen, die ausgestattet mit einem geeigneten Ventil in einem geeigneten Inhalator zur Anwendung gelangen können und die eine der vorstehend genannten erfindungsgemäßen treibgashaltigen Inhalationsaerosole enthalten. Geeignete Kartuschen und Verfahren zur Abfüllung dieser Kartuschen mit den erfindungsgemäßen treibgashaltigen Inhaltionsaerosolen sind aus dem Stand der Technik bekannt.

### C) Treibgasfreie Inhaltionslösungen:

Die erfindungsgemäßen Verwendung von Tiotropiumsalzen 1 erfolgt bevorzugt zur Herstellung von treibgasfreien Inhalationslösungen und Inhaltionssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die 1 enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Erfindungsgemäß kann auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg /100 ml, bevorzugt unter 50 mg/100 ml, besonders beovorzugt unter 20 mg/ 100ml. Generell sind solche Inhaltionslösungen bevorzugt, in denen der Gehalt an Natriumedetat bei 0 bis 10mg/100ml liegt.

Den im Rahmen der erfindungsgemäßen Verwendung einsetzbaren treibgasfreien Inhaltionslösungen können Co-Solvention und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hitfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten. Konservierungsstoffe sind vorzugsweise in Konzentrationen von bis zu 50mg/100ml, besondersbevorzugt zwischen 5 und 20 mg/100ml enthalten.

Die im Rahmen der erfindungsgemäßen Verwendung einsetzbaren bevorzugten treibgasfreien Inhaltionslösungen enthalten außer dem Lösungsmittel Wasser und den Tiotropiumsalzen 1 nur noch Benzalkoniumchlorid und Natriumedetat. In einer anderen bevorzugten Ausführungsform wird auf Natriumedetat verzichtet.

Zur Applikation der im Rahmen der erfindungsgemäßen Verwendung einsetzbaren treibgasfreien Inhaltionslösungen sind besonders solche Inhalatoren geeignet, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 µL, bevorzugt weniger als 50 µL, besonders bevorzugt zwischen 10 und 30 µL Wirkstofflösung mit bevorzugt einem Hub zu einem Aerosol mit einer durchschnittlichen Teilchengröße von weniger als 20 µm, bevorzugt weniger als 10 µm, so vernebelt werden können, daß der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der intemationalen Patentanmeldung WO 91/14468 als auch in der WO 97/12687 (dort insbesondere Figuren 6a und 6b) ausführlich beschrieben. Die dort beschriebenen Vernebler (Devices) sind auch unter der Bezeichnung Respimat® bekannt.

Dieser Vernebler (Respimat® ) kann vorteilhaft zur Erzeugung der erfindungsgemäßen inhalierbaren Aerosole enthaltend die Tiotropiumsalze 1 eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so daß inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-94107607 offenbart; auf diese Schrift wird hiermit inhaltlich Bezug genommen, insbesondere auf die dort offenbarte Figur 1 und deren Beschreibung.
Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen.

Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchen- bzw. Tröpfchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

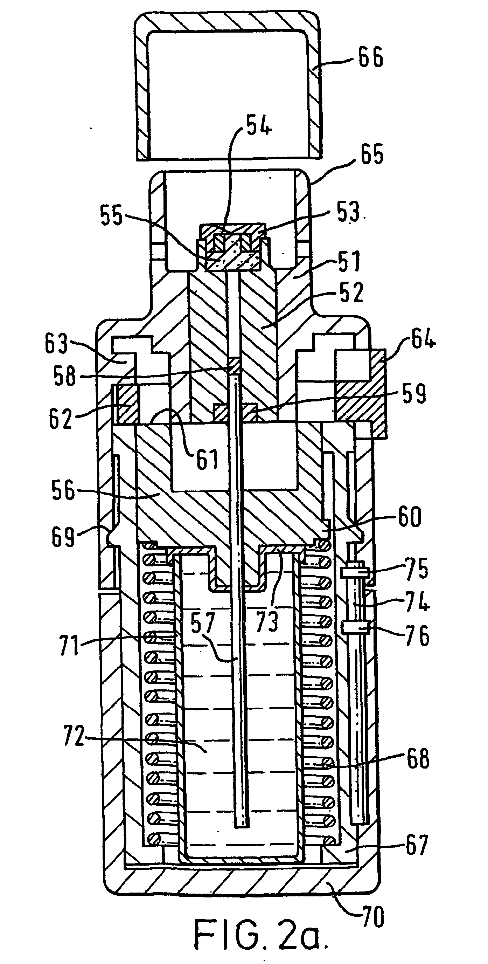

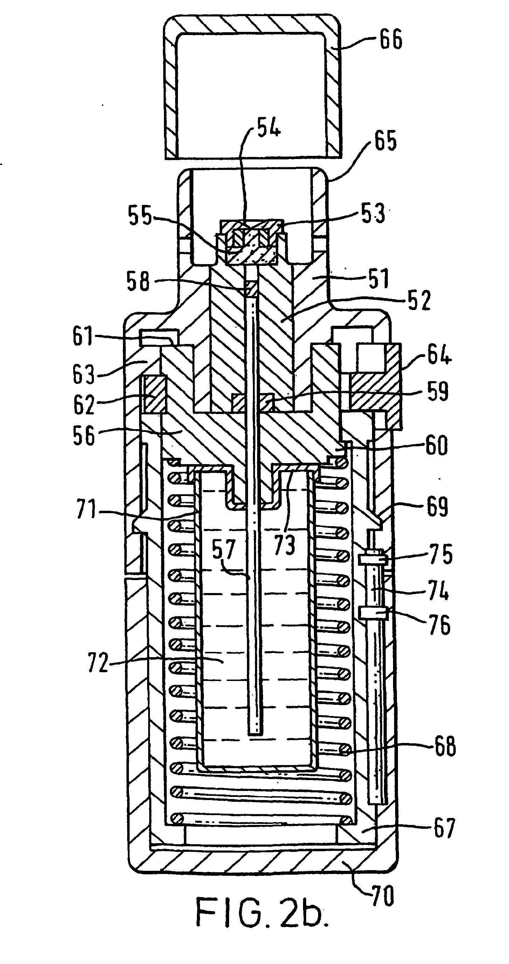

In den dieser Patentanmeldung beigefügten Figuren 2a/b, die identisch sind mit den Figuren 6 a/b der WO 97/12687, ist der Vernebler (Respimat®) beschrieben, mit dem die erfindungsgemäßen wäßrigen Aerosolzubereitungen vorteilhaft inhaliert werden können.

Figur 2a zeigt einen Längsschnitt durch den Zerstäuber bei gespannter Feder, Figur 2b zeigt einen Längsschnitt durch den Zerstäuber bei entspannter Feder.

Das Gehäuseoberteil (51) enthält das Pumpengehäuse (52), an dessen Ende-der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die im Rahmen der erfindungsgemäßen Verwendung einsetzbaren Aerosolzubereitungen zu einem für die Inhalation geeignetem Aerosol zu vernebeln.

Werden die im Rahmen der erfindungsgemäßen Verwendung einsetzbaren treibgasfreien Inhalationslösungen mittels der vorstehend beschriebenen Technik (Respimat® ) vemebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die im Rahmen der erfindungsgemäßen Verwendung einsetzbaren treibgasfreien Inhalationslösungen können jedoch auch mittels anderer als der vorstehend beschriebenen Inhalatoren, beispielsweise Jet-Stream-Inhalatoren oder anderen stationären Vemeblem vernebelt werden.

Dementsprechend betrifft ein weiterer Aspekt der vorliegenden Erfindung die erfindungsgemäße Verwendung von Tiotropiumsalzen 1 zur Herstellung eines Arzneimittels in Form von wie vorstehend beschriebenen treibgasfreien Inhaltionslösungen oder Suspensionen in Verbindung mit einer zur Verabreichung dieser Formulierungen geeigneten Vorrichtung, bevorzugt in Verbindung mit dem Respimat®. Bevorzugt zielt die vorliegende Erfindung auf die im Rahmen der erfindungsgemäßen Verwendung von 1 zur Herstellung von treibgasfreien Inhaltionslösüngen oder Suspensionen gekennzeichnet durch einen Gehalt an 1 in Verbindung mit der unter der Bezeichnung Respimat® bekannten Vorrichtung. Ferner betrifft die vorliegende Erfindung die erfindungsgemäße Verwendung vorstehend genannter Vorrichtungen zur Inhalation, bevorzugt den Respimat®, dadurch gekennzeichnet, daß sie vorstehend beschriebene erfindungsgemäße treibgasfreie Inhaltionslösungen oder Suspensionen enthalten.

Die im Rahmen der erfindungsgemäßen Verwendung von 1 einsetzbaren treibgasfreien Inhalationslösungen oder Suspensionen können neben den vorstehend, zur Applikation im Respimat vorgesehenen Lösungen und Suspensionen auch als Konzentrate oder sterile gebrauchsfertige Inhalationslösungen bzw. -suspensionen vorliegen. Aus den Konzentraten lassen sich beispielsweise durch Zugabe von isotonischen Kochsalzlösungen gebrauchsfertige Formulierungen generieren. Sterile gebrauchsfertige Formulierungen können mittels energiebetriebener Stand- oder transportabler Vemebler, die inhalierbare Aerosole mittels Ultraschall oder Druckluft nach dem Venturiprinzip oder anderen Prinzipien erzeugen, appliziert werden.

Dementsprechend betrifft ein weiterer Aspekt der vorliegenden Erfindung die erfindungsgemäße Verwendung von 1 in Form von wie vorstehend beschriebenen treibgasfreien Inhaltionslösungen oder Suspensionen, die als Konzentrate oder sterile gebrauchsfertige Formulierungen vorliegen, in Verbindung mit einer zur Verabreichung dieser Lösungen geeigneten Vorrichtung, dadurch gekennzeichnet, daß es sich bei dieser Vorrichtung um einen energiebetriebenen Stand- oder transportablen Vernebler handelt, der inhalierbare Aerosole mittels Ultraschall oder Druckluft nach dem Venturiprinzip oder anderen Prinzipien erzeugt.

Die folgenden Beispiele dienen einer weitergehenden Erläuterung der vorliegenden Erfindung,

### Ausgangsmaterialien

### Tiotropiumbromid:

Das in den nachfolgenden Formulierungsbeispielen eingesetzte Tiotropiumbromid kann wie in der Europäischen Patentanmeldung EP 418 716 A1 beschrieben, erhalten werden.

Zur Herstellung der erfindungsgemäßen Inhalationspulver kann ebenfalls kristallines Tiotropiumbromidmonohydrat eingesetzt werden. Dieses kristalline Tiotropiumbromidmonohydrat ist gemäß nachfolgend beschriebener Vorgehensweise erhältlich.
In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet. Ausbeute : 13,4 kg Tiotropiumbromidmonohydrat (86 % d. Th.)

Das so erhaltene kristalline Tiotropiumbromidmonohydrat wird nach bekannten Verfahren mikronisiert, um den Wirkstoff in Form der mittleren Teilchengröße bereitzustellen, die den erfindungsgemäßen Spezifikationen entspricht.

### Formulierungsbeispiele

A) Inhaltionspulver:
1)

| **Bestandteile** | **µg pro Kapsel** |
|---|---|
| Tiotropiumbromid | 10,8 |
| Lactose | 4989,2 |
| Summe | 5000 |

2)

| **Bestandteile** | **µg pro Kapsel** |
|---|---|
| Tiotropiumbromid | 21,7 |
| Lactose | 4978,3 |
| Summe | 5000 |

3)

| **Bestandteile** | **µg pro Kapsel** |
|---|---|
| Tiotropiumbromid x H₂O | 22,5 |
| Lactose | 4977,5 |
| Summe | 5000 |

B) Treibgashaltige Inhaltionsaerosole:
1) Suspensionsaerosol:

| **Bestandteile** | **Gew-%** |
|---|---|
| Tiotropiumbromid | 0,015 |
| Sojalecithin | 0,2 |
| TG 134a : TG227 = 2:3 | ad 100 |

2) Suspensionsaerosol:

| **Bestandteile** | **Gew-%** |
|---|---|
| Tiotropiumbromid | 0,029 |
| Ethanol, absolut | 0,5 |
| Isopropylmyristat | 0,1 |
| TG 227 | ad 100 |

3) Lösungsaerosol:

| **Bestandteile** | **Gew-%** |
|---|---|
| Tiotropiumbromid | 0,042 |
| Ethanol, absolut | 30 |
| Wasser, gereinigt | 1,5 |
| Zitronensäure, wasserfrei | 0,002 |
| TG 134a | ad 100 |

C) Treibgasfreie Inhaltionslösunqen:
1) Lösung zur Anwendung im Respimat®:

| **Bestandteile** | **mg/100mL** |
|---|---|
| Tiotropiumbromid | 148,5 |
| Benzalkoniumchlorid | 10 |
| Natriumedetat | 10 |
| Salzsäure (aq) | ad pH 2,9 |
| Wasser | ad 100mL |

Verwendung der Lösung im Respimat führt zu einer Dosierung von 10µg pro Dosierung 1.
2) Lösung zur Anwendung im Respimat®:

| **Bestandteile** | **mg/100mL** |
|---|---|
| Tiotropiumbromid | 148,5 |
| Benzalkoniumchlorid | 10 |
| Salzsäure (aq) | ad pH 2,9 |
| Wasser | ad 100mL |

Verwendung der Lösung im Respimat führt zu einer Dosierung von 10µg pro Dosierung 1.
3) Lösung zur Anwendung im Respimat®:

| **Bestandteile** | **mg/100mL** |
|---|---|
| Tiotropiumbromid | 297,1 |
| Benzalkoniumchlorid | 10 |
| Natriumedetat | 10 |
| Salzsäure (aq) | ad pH 2,9 |
| Wasser | ad 100mL |

Verwendung der Lösung im Respimat führt zu einer Dosierung von 20µg pro Dosierung 1 und 25µg/Dosierung 2.
4) Lösung zur Anwendung im Respimat®:

| **Bestandteile** | **mg/100mL** |
|---|---|
| Tiotropiumbromid | 297,1 |
| Benzalkoniumchlorid | 10 |
| Salzsäure (aq) | ad pH 2,9 |
| Wasser | ad 100mL |

Verwendung der Lösung im Respimat führt zu einer Dosierung von 20µg pro Dosierung 1.
5) Lösung zur Anwendung im Respimat® :

| **Bestandteile** | **mg/100mL** |
|---|---|
| Tiotropiumbromid | 148,5 |
| Benzalkoniumchlorid | 8 |
| Natriumedetat | 50 |
| Salzsäure (aq) | ad pH 2,5 |
| Wasser | ad 100mL |

Verwendung der Lösung im Respimat führt zu einer Dosierung von 10µg pro Dosierung 1.
6) Lösung zur Anwendung im Respimat® :

| **Bestandteile** | **mg/100mL** |
|---|---|
| Tiotropiumbromid | 1,5 |
| Benzalkoniumchlorid | 8 |
| Natriumedetat | 10 |
| Salzsäure (aq) | ad pH 2,5 |
| Wasser | ad 100mL |

Verwendung der Lösung im Respimat führt zu einer Dosierung von 0,1µg pro Dosierung 1.
7) Lösung zur Anwendung im Respimat® :

| **Bestandteile.** | **mg/100mL** |
|---|---|
| Tiotropiumbromid | 14,9 |
| Benzalkoniumchlorid | 10 |
| Natriumedetat | 50 |
| Salzsäure (aq) | ad pH 3,5 |
| Wasser | ad 100mL |

Verwendung der Lösung im Respimat führt zu einer Dosierung von 1 µg pro Dosierung 1.
8) Konzentrierte Lösung:

| **Bestandteile** | **mg/100mL** |
|---|---|
| Tiotropiumbromid | 1486,1 |
| Benzalkoniumchlorid | 20 |
| Natriumedetat | 100 |
| Salzsäure (aq) | ad pH 3,5 |
| Wasser | ad 100mL |

## Patentansprüche

1. Verwendung von Tiotropiumsalzen zur Herstellung eines Arzneimittels zur Vorbeugung oder Behandlung von zystischer Fibrose, idiopathischer Lungenfibrose oder fibrosierender Alveolitis, **dadurch gekennzeichnet, daß** es sich bei den Tiotropiumsalze um Verbindungen handelt, die neben Tiotropium als Gegenion (Anion) Chlorid, Bromid, lodid, Methansulfonat, para-Toluolsulfonat oder Methylsulfat enthalten.

2. Verwendung nach Anspruch 1 **dadurch gekennzeichnet, daß** es sich bei den Tiotropiumsalzen um Verbindungen handelt, die neben Tiotropium als Gegenion Methansulfonat, Chlorid, Bromid oder lodid, bevorzugt Methansulfonat oder Bromid enthalten.

3. Verwendung nach einem der Ansprüche 1 bis 2, **dadurch gekennzeichnet, daß** das Arzneimittel in Form einer für die Inhalation geeigneten Darreichungsform vorliegt.

4. Verwendung nach Anspruche 3, **dadurch gekennzeichnet, daß** es sich bei der für die Inhalation geeigneten Darreichungsform um eine Darreichungsform, ausgewählt aus der Gruppe Inhalationspulver, treibgashaltige Dosieraerosole und treibgasfreie Inhalationslösungen, andelt.

5. Verwendung nach Anspruch 4, **dadurch gekennzeichnet, daß** die Darreichungsform ein Inhalationspulver ist, welches die beanspruchten Tiotropiumsalze im Gemisch mit geeigneten physiologisch unbedenklichel-N Hilfsstoffen, ausgewählt aus der Gruppe bestehend aus Monosaccharide-N, Disaccharide N, Oligo- und Polysaccharide-N, Polyalkohole-N, Salze-N, oder Mischungen dieser Hilfsstoffe miteinander, enthält.

6. Verwendung nach Anspruch 4, **dadurch gekennzeichnet, daß** es sich bei der Darreichungsform um ein treibgashaltiges Inhalationsaerosol handelt, welches die beanspruchtenTiotropiumsalze in gelöster oder dispergierter Form enthält.

7. Verwendung nach Anspruch 6, **dadurch gekennzeichnet, daß** das treibgashaltige Inhalationsaerosol als Treibgas Kohlenwasserstoffe, wie n-Propan, n-Butan oder Isobutan oder Halogenkohlenwasserstoffe, wie fluorierte Derivate des Methans, Ethans, Propans, Butans, Cyclopropans oder Cyclobutans enthält.

8. Verwendung nach Anspruch 6 oder 7, **dadurch gekennzeichnet, daß** das treibgashaltige Inhalationsaerosol als Treibgas TG134a, TG227 oder Gemische davon enthält.

9. Verwendung nach Anspruche 6, 7 oder 8, **dadurch gekennzeichnet, daß** das treibgashaltige Inhalationsaerosol gegebenenfalls einen oder mehrere weitere Bestandteile ausgewählt aus der Gruppe bestehend aus Kosolventien, Stabilisatoren, oberflächenaktive-N Mittel-N (surfactants), Antioxidantien, Schmiermittel-N und Mittel-N zur Einstellung des pH-Werts enthält.

10. Verwendung nach Anspruch 4, **dadurch gekennzeichnet, daß** es sich bei der Darreichungsform um eine treibgasfreie Inhalationslösung handelt, die als Lösemittel Wasser, Ethanol oder ein Gemisch aus Wasser und Ethanol enthält.

11. Verwendung nach Anspruch 10, **dadurch gekennzeichnet, daß** der pH der Lösung 2-7, bevorzugt 2 - 5 beträgt.

12. Verwendung nach Anspruch 10 oder 11 **dadurch gekennzeichnet, daß** die Inhalationslösung gegebenenfalls weitere Co-Solventien und/oder Hilfsstoffe enthält.

13. Verwendung nach Anspruch 12, **dadurch gekennzeichnet, daß** die Inhalationslösung als Co-Solventien Bestandteile enthält, die Hydroxylgruppen oder andere polare Gruppen enthalten, beispielsweise Alkohole- insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

14. Verwendung nach Anspruch 12 oder 13, **dadurch gekennzeichnet, daß** die Inhalationslösung, als Hilfsstoffe oberflächenaktive Stoffe Stabilisatoren, Komplexbildner, Antioxidantien und/oder Konservierungsstoffe, Geschmackstoffe, pharmakologisch unbedenkliche Salze und/oder Vitamine enthält.

15. Verwendung nach Anspruch 14, **dadurch gekennzeichnet, daß** die Inhalationslösung als Komplexbildner Editinsäure oder ein Salz der Editinsäure, bevorzugt Natriumedetat, enthält.

16. Verwendung nach einem der Ansprüche 10 bis 15, **dadurch gekennzeichnet, daß** die. Inhalationslösung neben den Wirkstoffen und dem Lösemittel nur noch Bezalkoniumchlorid und Natriumedetat enthält.

17. Verwendung nach einem der Ansprüche 10 bis 16, **dadurch gekennzeichnet, daß** es sich bei der Inhalationslösung um ein Konzentrat oder eine sterile gebrauchsfertige Inhalationslösung handelt.

## Claims

1. Use oftiotropium salts for preparing a pharmaceutical composition for the prevention or treatment of cystic fibrosis, idiopathic lung fibrosis or fibrosing alveolitis, **characterised in that** the tiotropium salts are compounds which contain in addition to tiotropium as counter-ion (anion) chloride, bromide, iodide, methanesulphonate, para-toluenesulphonate or methylsulphate.

2. Use according to claim 1, **characterised in that** the tiotropium salts are compounds which contain in addition to tiotropium as counter-ion methanesulphonate, chloride, bromide or iodide, preferably methanesulphonate or bromide.

3. Use according to one of claims 1 and 2, **characterised in that** the pharmaceutical composition is presented in the form of a formulation suitable for inhalation.

4. Use according to claim 3, **characterised in that** the formulation suitable for inhalation is a formulation selected from among powders for inhalation, metered-dose aerosols containing propellant gas and propellant-gas-free inhalable solutions.

5. Use according to claim 4, **characterised in that** the formulation is an inhalable powder which contains the tiotropium salts claimed, in admixture with suitable physiologically acceptable excipients selected from among monosaccharides, disaccharides, oligo- and polysaccharides, polyalcohols, salts or mixtures of these excipients with one another.

6. Use according to claim 4, **characterised in that** the formulation is an inhalable aerosol containing propellant gas which contains the tiotropium salts claimed in dissolved or dispersed form.

7. Use according to claim 6, **characterised in that** the inhalable aerosol containing propellant gas contains as propellant gas hydrocarbons such as n-propane, n-butane or isobutane or halohydrocarbons such as fluorinated derivatives of methane, ethane, propane, butane, cyclopropane or cyclobutane.

8. Use according to claim 6 or 7, **characterised in that** the inhalable aerosol containing propellant gas contains as propellant gas TG134a, TG227 or mixtures thereof.

9. Use according to claim 6, 7 or 8, **characterised in that** the inhalable aerosol containing propellant gas optionally contains one or more other ingredients selected from among co-solvents, stabilisers, surfactants, antioxidants, lubricants and pH adjusters.

10. Use according to claim 4, **characterised in that** the formulation is a propellant-free inhalable solution which contains as solvent water, ethanol or a mixture of water and ethanol.

11. Use according to claim 10, **characterised in that** the pH of the solution is 2 - 7, preferably 2 - 5.

12. Use according to claim 10 or 11, **characterised in that** the inhalable solution optionally contains other co-solvents and/or excipients.

13. Use according to claim 12, **characterised in that** the inhalable solution contains as co-solvents ingredients which contain hydroxyl groups or other polar groups, e.g. alcohols-particularly isopropyl alcohol, glycols-particularly propyleneglycol, polyethyleneglycol, polypropyleneglycol, glycolether, glycerol, polyoxyethylene alcohols and polyoxyethylene fatty acid esters.

14. Use according to claim 12 or 13, **characterised in that** the inhalable solution contains as excipients surfactants, stabilisers, complexing agents, antioxidants and/or preservatives, flavourings, pharmacologically acceptable salts and/or vitamins.

15. Use according to claim 14, **characterised in that** the inhalable solution contains as complexing agent editic acid or a salt of editic acid, preferably sodium edetate.

16. Use according to one of claims 10 to 15, **characterised in that** the inhalable solution contains only benzalkonium chloride and sodium edetate in addition to the active substances and the solvent.

17. Use according to one of claims 10 to 16, **characterised in that** the inhalable solution is a concentrate or a sterile inhalable solution ready for use.

## Revendications

1. Utilisation de sels de tiotropium pour la production d'un médicament pour la prévention ou le traitement de la fibrose kystique, de la fibrose pulmonaire idiopathique ou de l'alvéolite fibrosante **caractérisée en ce que** les sels de tiotropium sont des composés qui contiennent, outre du tiotropium, comme contre-ion (anion) du chlorure, bromure, iodure, méthanesulfonate, paratoluènesulfonate ou méthylsulfate.

2. Utilisation selon la revendication 1 **caractérisée en ce que** les sels de tiotropium sont des composés qui contiennent, outre du tiotropium, comme contre-ion du méthanesulfonate, chlorure, bromure ou iodure, de préférence du méthanesulfonate ou du bromure.

3. Utilisation selon l'une des revendications 1 à 2 **caractérisée en ce que** le médicament est sous forme d'une forme d'administration appropriée à l'inhalation.

4. Utilisation selon la revendication 3 **caractérisée en ce que** la forme d'administration appropriée à l'inhalation est une forme d'administration choisie dans le groupe poudre pour inhalation, aérosols de dosage contenant un gaz propulseur et solutions pour inhalation sans gaz propulseur.

5. Utilisation selon la revendication 4 **caractérisée en ce que** la forme d'administration est une poudre pour inhalation qui contient les sels de tiotropium revendiqués en mélange avec des adjuvants physiologiquement acceptables appropriés choisis dans le groupe consistant en les monosaccharides, les disaccharides, les oligo- et polysaccharides, les polyalcools, les sels ou des mélanges de ces adjuvants.

6. Utilisation selon la revendication 4 **caractérisée en ce que** la forme d'administration est un aérosol pour inhalation contenant un gaz propulseur qui contient les sels de tiotropium revendiqués sous forme dissoute ou dispersée.

7. Utilisation selon la revendication 6 **caractérisée en ce que** l'aérosol pour inhalation contenant un gaz propulseur contient comme gaz propulseur des hydrocarbures, comme le n-propane, le n-butane ou l'isobutane ou des halogénohydrocarbures, comme des dérivés fluorés du méthane, de l'éthane, du propane, du butane, du cyclopropane ou du cyclobutane.

8. Utilisation selon la revendication 6 ou 7 **caractérisée en ce que** l'aérosol pour inhalation contenant un gaz propulseur contient comme gaz propulseur TG134a, TG227 ou des mélanges de ceux-ci.

9. Utilisation selon la revendication 6, 7 ou 8 **caractérisée en ce que** l'aérosol pour inhalation contenant un gaz propulseur contient éventuellement un ou plusieurs autres constituants choisis dans le groupe consistant en les cosolvants, les stabilisants, les agents tensioactifs (surfactants), les antioxydants, les lubrifiants et les agents pour régler le pH.

10. Utilisation selon la revendication 4 **caractérisée en ce que** la forme d'administration est une solution pour inhalation sans gaz propulseur qui contient comme solvant de l'eau, de l'éthanol ou un mélange d'eau et d'éthanol.

11. Utilisation selon la revendication 10 **caractérisée en ce que** le pH de la solution est 2 - 7, de préférence 2 - 5.

12. Utilisation selon la revendication 10 ou 11 **caractérisée en ce que** la solution pour inhalation contient éventuellement d'autres co-solvants et/ou adjuvants.

13. Utilisation selon la revendication 12 **caractérisée en ce que** la solution pour inhalation contient comme co-solvants des constituants qui contiennent des groupes hydroxyle ou d'autres groupes polaires, par exemple des alcools - en particulier l'alcool isopropylique, des glycols - en particulier le propyléneglycol, le polyéthylèneglycol, le polypropylèneglycol, un éther de glycol, le glycérol, des alcools polyoxyéthylénés et des esters de polyoxyéthylène-acides gras.

14. Utilisation selon la revendication 12 ou 13 **caractérisée en ce que** la solution pour inhalation contient comme adjuvants des substances tensioactives, des stabilisants, des complexants, des antioxydants et/ou des conservateurs, des agents de sapidité, des sels pharmacologiquement acceptables et/ou des vitamines.

15. Utilisation selon la revendication 14 **caractérisée en ce que** la solution pour inhalation contient comme complexant l'acide édétique ou un sel d'acide édétique, de préférence l'édétate de sodium.

16. Utilisation selon l'une des revendications 10 à 15 **caractérisée en ce que** la solution pour inhalation ne contient, outre les principes actifs et le solvant, que du chlorure de benzalkonium et de l'édétate de sodium.

17. Utilisation selon l'une des revendications 10 à 16 **caractérisée en ce que** la solution pour inhalation est un concentré ou une solution pour inhalation prête à l'emploi stérile.
